# EUROPEAN PATENT APPLICATION

(11) **EP 1 752 096 A2**
(43) Date of publication of application: **14.02.2007**
(21) Application number: 06254203.0
(22) Date of filing: 10.08.2006
(51) Int. Cl.: A61B 5/15, A61B 10/00

(54) **Sampling module for extracting interstitial fluid**

(30) Priority: 11.08.2005 US 203022
(71) Applicant: LifeScan, Inc., Milpitas, CA 95035 (US)
(72) Inventor: Mechelke, Joel, Stillwater, Minnesota 55082 (US); Rademacher, Thomas C., St. Paul, Minnesota 55104 (US); Hilgers, Michael Edward, Lake Elmo, Minnesota 55042 (US); Stout, Phil, Roseville, Minnesota 55113 (US)
(74) Representative: Tunstall, Christopher Stephen

(57) **Abstract**

A sampling module for extracting interstitial fluid (ISF) from a user's body includes a housing assembly and a pressure ring assembly operatively contained within the housing assembly. The pressure ring assembly includes a penetration member configured for penetrating a target site of the user's body and, subsequently, residing in the target site and extracting ISF therefrom. The pressure ring assembly also includes a pressure ring configured for applying pressure to the user's body in a first vicinity of the target site and a floating ring with an adhesive layer disposed on it's distal end. The adhesive layer of the sampling module provides for adhesive attachment of the floating ring to the user's body in a second vicinity of the target site, thereby influencing target site deformation during use of the sampling module.

## Description

### BACKGROUND OF THE INVENTION

1. Field of the Invention

The present invention relates, in general, to medical devices and their associated methods and, in particular, to devices and methods for extracting interstitial fluid.

2. Description of the Related Art

In recent years, efforts related to medical devices for monitoring analytes (e.g., glucose) in bodily fluids (e.g., blood and interstitial fluid [ISF]) have been directed toward developing devices and methods that facilitate continuous or semi-continuous monitoring and toward simplifying such devices and methods. Simplification of such devices and methods enable users to self-monitor analytes at home or in other convenient locations.

In the context of blood glucose monitoring, continuous or semi-continuous monitoring devices and methods are advantageous in that they provide enhanced insight into blood glucose concentration trends, the effect of food and medication on blood glucose concentration and a user's overall glycemic condition. In practice, however, continuous and semi-continuous monitoring devices can have drawbacks. For example, during extraction of an interstitial fluid (ISF) sample from a target site (e.g., a target site in a user's skin layer), ISF flow rate may decay over time. Furthermore, continuous and semi-continuous devices may be relatively large and, therefore, inconvenient and uncomfortable to wear.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
FIG. 1 is a simplified perspective view of a sampling module for extracting interstitial fluid (ISF) according to an exemplary embodiment of the present invention;
FIG. 2 is a simplified exploded perspective view of a housing assembly of the sampling module of FIG. 1;
FIG. 3 is a simplified exploded perspective view of a pressure ring assembly of the sampling module of FIG. 1;
FIG. 4 is a simplified bottom plan view a floating ring of the sampling module of FIG. 1;
FIG. 5 is a simplified cross-sectional side view of the floating ring of FIG. 4 taken through line A-A of FIG. 4;
FIG. 6 is a simplified cross-sectional side view of the pressure ring of the sampling module of FIG. 1;
FIG. 7 is a simplified cross-sectional depiction of the sampling module of FIG. 1 attached to a user's body during use with the pressure ring and floating ring thereof in a retracted state;
FIG. 8 is a simplified cross-sectional depiction of the sampling module of FIG. I attached to a user's body during use with the pressure ring and floating ring thereof in a deployed state;
FIG. 9 is a simplified cross-sectional view of a sampling module for extracting ISF, that does not include a floating ring, in a retracted state;
FIG. 10 is a simplified cross-sectional view of a sampling module for extracting ISF, that does not include a floating ring, in a deployed state;
FIG. 11 is a simplified cross-sectional view of a sampling module for extracting ISF according to another exemplary embodiment of the present invention in a deployed state; and
FIG. 12 is a flow diagram depicting stages in a process for extracting interstitial fluid according to an exemplary embodiment of the present invention.

### DETAILED DESCRIPTION OF PREFERRED EMBODIMENTS

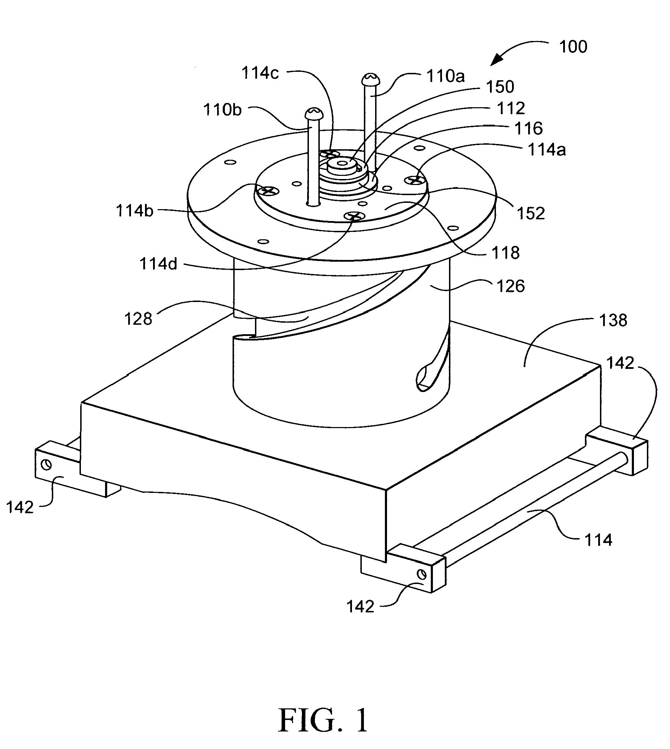

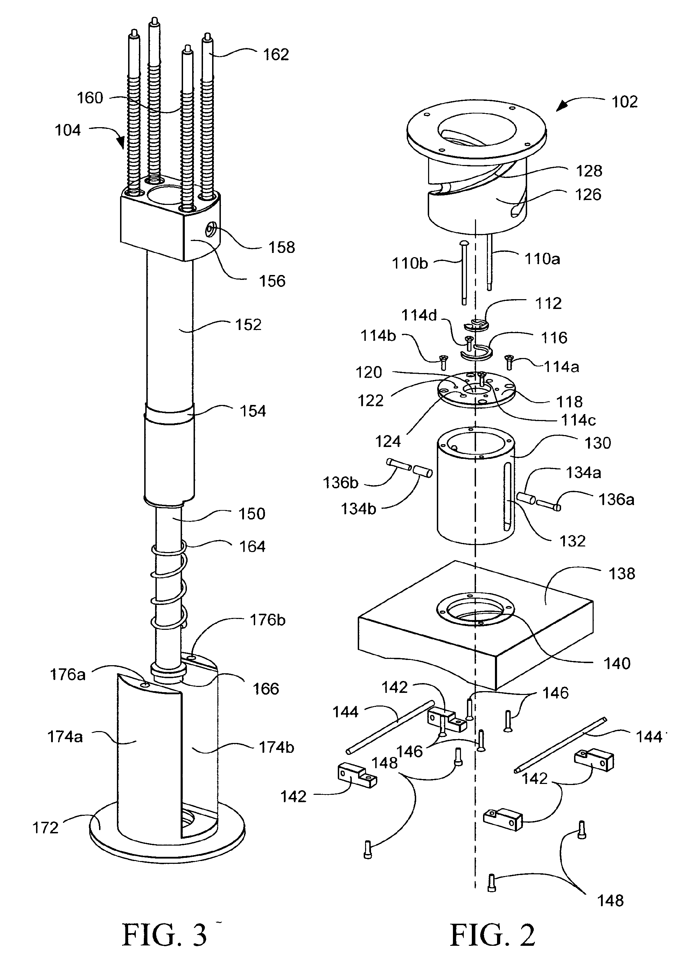

FIGs. 1, 2 and 3 are simplified perspective views of a sampling module 100 and assemblies thereof for extracting interstitial fluid (ISF) from a user's body according to an exemplary embodiment of the present invention. It should be noted that FIGs. 1-3 are not necessarily drawn to an identical scale. Sampling module 100 includes a housing assembly 102 (see FIG. 2 in particular) and a pressure ring assembly 104 (see FIG. 3 in particular) operatively contained within housing assembly 102.

Referring to FIG. 2, housing assembly 102 includes rods 110a and 110b, an inner E-Ring 112, screws 114a, 114b and 114c, an outer E-ring 116, a cap 118 (with cap opening 120, first holes 122 and second holes 124 therethrough), a cam 126 (with helical groove 128 therein), a ring housing 130 (with a channel 132 therein), rollers 134 and screws 136a and 136b. Housing assembly 102 also includes a plate 138 (with plate opening 140 therethrough), brackets 142, pins 144, screws 146 and screws 148.

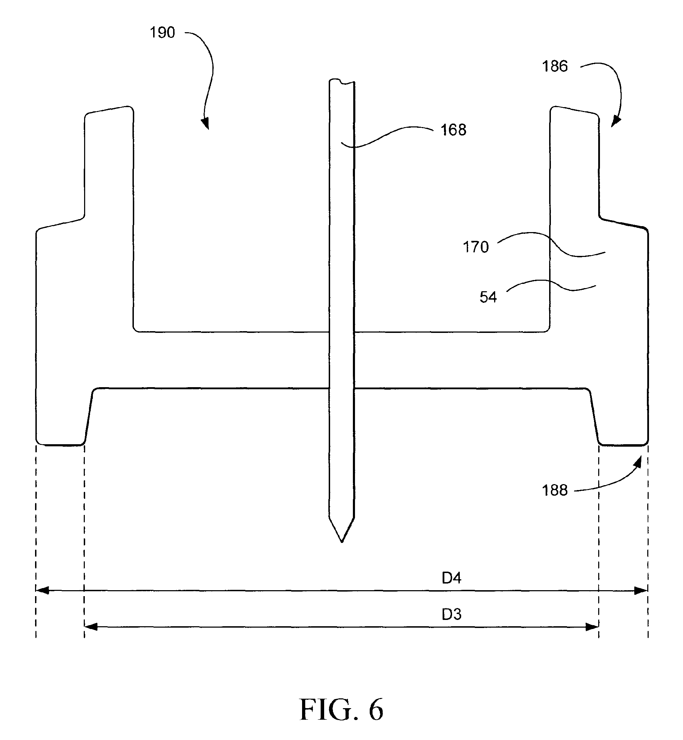

Referring in particular to FIG. 3, pressure ring assembly 104 includes an inner shaft 150, an outer shaft 152 (with shaft step 154), a spring retainer 156 (with spring retainer recess 158 therein), pressure springs 160 and poles 162. Pressure ring assembly 104 also includes penetration spring 164 (disposed in a concentric manner with respect to inner shaft 150), penetration member carriage 166 (which includes a penetration member 168, not depicted in FIGs. 1-3 but shown in FIGs. 6, 7 and 8), pressure ring 170 (not depicted in FIGs. 1-3 but illustrated in FIGs. 6, 7 and 8) and floating ring 172 (with floating ring arms 174a and 174b and holes 176a and 176b).

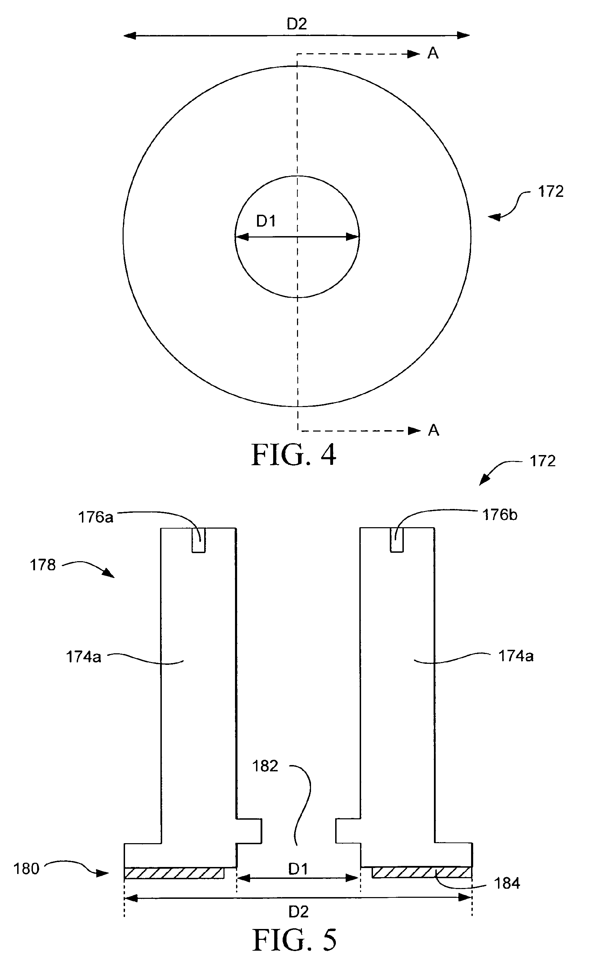

FIGs. 4 and 5 are a simplified bottom plan view and a cross-sectional side view, respectively, of floating ring 172. Floating ring 172 has a proximal end 178, a distal end 180 and a floating ring opening 182. As depicted in FIGs. 4 and 5, floating ring opening 182 has a diameter of D1 (i.e., an inner diameter) and floating ring 172 has an overall diameter of D2 (i.e., an outer diameter).

Floating ring 172 also includes an adhesive layer 184 disposed on distal end 180. Adhesive layer 184 can be formed, for example, from double-sided pressure sensitive acrylic-based adhesive material, including such material as is commercially available from 3M Corp as product number 9889.

Adhesive layer 184 provides for floating adhesive attachment of floating ring 172 to the user's body in a vicinity of a target site, thereby influencing target site deformation during use of sampling module 100. For example, the floating ring can influence target site deformation by placing the target site under tension (i.e., holding the target site and the area between the floating ring and the target site taut) during application of pressure to the user's body by pressure ring 170. This beneficial function of floating rings employed in sampling modules according to embodiments of the present invention is described further below.

The proximal end of floating ring 172 is "floatably" connected to housing assembly 102. More specifically, on the uppermost portion of floating ring arms 174a and 174b, holes 176a and 176b thereof are configured to loosely and slidably engage with rods 110a and 110b of housing assembly 102. However, rods 110a and 110b are constrained via interaction with second holes 124 of cap 118. Therefore, floating ring 172 is free to move (i.e., "float") vertically, but not rotationally.

Since floating ring 172 is free to float in the vertical direction, it does not apply any downward pressure in the vicinity of the target site other than a negligible pressure due solely to the mass of the floating ring and pressure resulting from the mass of other sampling module components that may be essentially transferred to the floating ring (and hence to the target site) by frictional forces. In other words, the pressure is due to the mass of the pressure ring and pressure related to mass of the sampling module that is transferred to the vicinity of the target site via the floating ring. The "floating" nature of the floating ring is, therefore, beneficial in terms of minimizing the pressure applied to a user's body in the vicinity of a target site when a sampling module is in a retracted state.

FIG. 6 is a simplified cross-sectional side view of pressure ring 170 and a portion of penetration member 168. The penetration member of sampling modules according to various embodiments of the present invention can be, for example, a 25 gauge, hollow stainless steel needle with a bent tip, wherein a fulcrum for the tip bend is disposed between the needle's tip and the needle's heel. The penetration member can be formed, for example, of an injected molded plastic. Suitable needles for use as penetration members according to the present invention are described in U.S. Patent No. 6,702,791 and U.S. Application No. 10/185,605 (published as US 2003/0060784), which are hereby fully incorporated by reference.

Pressure ring 170 has a proximal end 186, a distal end 188 and a pressure ring opening 190 therethrough. Furthermore, pressure ring opening 190 has a diameter at the distal end of pressure ring 170 of D3 (i.e., an inner diameter) and the distal end diameter of pressure ring 170 itself is D4 (i.e., an outer diameter, see FIG. 6).

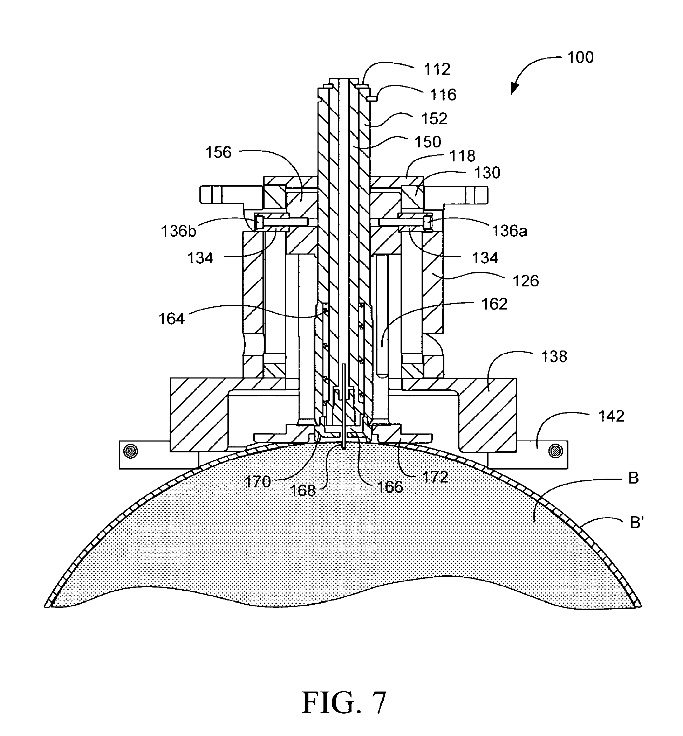

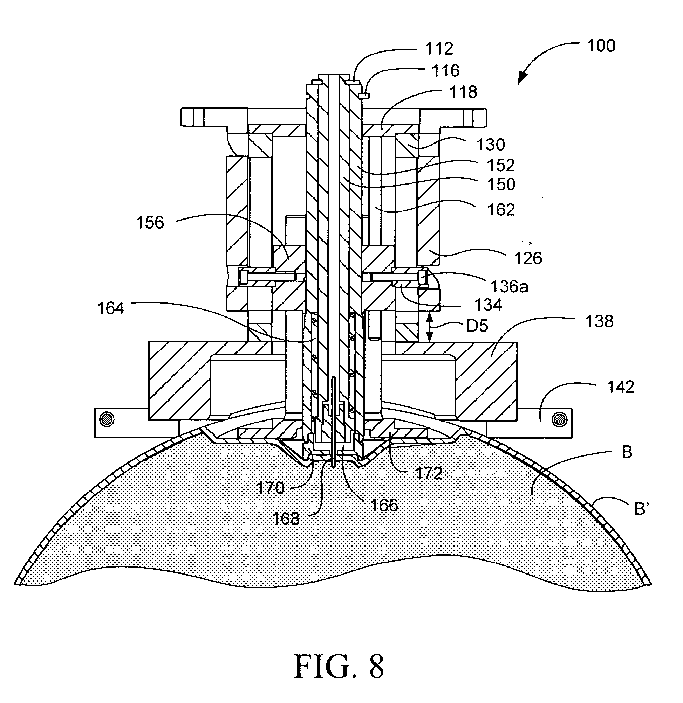

Diameter D3 of pressure ring 170 can range, for example, from about 4mm to about 12mm and diameter D4 of pressure ring 170 can range from about 5mm to about 13mm. The difference between diameter D4 and diameter D3 can range, for example, from about 1mm to about 9mm. Diameter D1 of floating ring 172 can, for example, range from about 7mm to about 18mm and diameter D2 can range, for example, from about 33mm to about 50mm. The difference between diameter D2 and diameter D1 can, for example, range from about 15 to about 43 millimeters. As is evident from FIGs. 7 and 8 described below, floating ring 172 is adhered at a second vicinity to the target site that is further from the target site than a first vicinity where the pressure ring applies pressure.

The operation of sampling module 100 and the interaction between various components thereof is described in additional detail below. From the descriptions, it will be evident that the penetration member of sampling modules according to various embodiments of the present invention is configured for penetrating a target site of a user's body (e.g., a skin layer of a user's forearm) and, subsequently, residing in the target site and extracting ISF therefrom.

In addition, it is also clear that the pressure ring of sampling modules according to various embodiments of the present invention is configured for applying pressure to the user's body in a first vicinity of the target site while said penetration member is residing the target site. Moreover, one skilled in the art will recognize that a detailed description of conventional components (e.g., screws and holes for receiving screws) has not been included herein in order to avoid obscuring various embodiments of the present invention.

FIG. 7 is a simplified cross-sectional depiction of sampling module 100 of FIG. 1 attached to a user's body (B) during use, with pressure ring 170 and floating ring 172 in a retracted state and penetration member 168 residing in a target site of the user's body. FIG. 8 is a simplified cross-sectional depiction of sampling module 100 attached to the user's body B during use with pressure ring 170 and floating ring 172 in a deployed state. In FIGs. 7 and 8, user's body B is depicted as including skin layer B'. Sampling module 100 can be attached to user's body B using, for example, a strap or armband (not shown in the FIGs.) attached to pins 144 of housing assembly 102.

Further details of the operation of sampling module 100 and the interaction of various components of sampling module 100 are described hereafter with reference to FIGs. 1 through 8. Cam 126 of housing assembly 102 is slidingly and concentrically disposed about ring housing 130. In addition, helical groove 128 of cam 126 is configured (i.e., "keyed") to operatively interact with roller 134. Ring housing 130 is attached to plate 138 via screws 146. In addition, channel 132 of ring housing 130 provides for vertical movement of roller 134.

Channel 132 and helical groove 128 operate such that roller 134 moves vertically upward (in the perspective of FIGs. 1-3, 7 and 8), when cam 126 is rotated in a clockwise direction and moves vertically downward when cam 126 is rotated a counterclockwise direction. Cam 126 can be caused to rotate by any suitable means including, for example, by a motor (not illustrated). Rollers 134 are configured to engage with screws 136a and 136b. In addition, screw 136a is bound within spring retainer recess 158 of spring retainer 156. This configuration provides for spring retainer 156 to move vertically upward when cam 126 is rotated in a clockwise direction and vertically downward with the counterclockwise rotation of cam 126. As described further below, the movement of spring retainer 156 is employed to place the sampling module (and thus pressure ring 170) in a retracted or deployed state. The use of two rollers 134 on opposing sides of the sampling module facilitates equalizing forces across the sampling module and avoiding inadvertent tilting of sampling module components.

Cap 118 of housing assembly 102 is generally disc-shaped, disposed on ring housing 130 and bound thereto by screws 114a and 114b. Cap opening 120 of cap 118 is of a sufficiently large diameter that a portion of outer shaft 152 can pass therethrough. Poles 162 guide pressure springs 160. An uppermost portion of each of poles 162 is fixedly mounted to first hole 122 of cap 118.

Pressure springs 160 are configured such that they exert an approximately constant force against spring retainer 156. In addition, pressure springs 160 can be configured such that they exert a relatively constant force over a range of spring compression lengths encountered during use of sampling module 100. For example, pressure springs 160 may apply a constant force ranging from about 2 Newtons to about 10 Newtons across a range of compression lengths from about 32 millimeters (uncompressed) to about 12 millimeters (compressed).

Spring retainer 156 is slidingly engaged with outer shaft 152 (see, for example, FIG. 3). Spring retainer 156 can be pushed in a downward direction until it reaches shaft step 154 of outer shaft 152. Once spring retainer 156 reaches shaft step 154, outer shaft 152 will also move in a downward direction if spring retainer 156 is further pushed downward.

Inner shaft 150 is concentrically and slidingly engaged with outer shaft 152 (see, for example, FIG. 3). Penetration member carriage 166 is attached to a distal end of inner shaft 150 (see FIG. 3). Penetration spring 164 is concentrically mounted on inner shaft 150, thereby providing for inner shaft 150 to be launched in a downward direction. Penetration spring 164 is retained on inner shaft 150 by the presence of outer shaft 152 and penetration member carriage 166. Penetration member 168 can be launched towards a target site by operative interaction of penetration member carriage 166, inner shaft 150 and penetration spring 164 at a velocity, for example, in the range of from about 3 meters/second to about 10 meters/second. However, since floating ring 172 (with adhesive layer 184) serves to hold the target site taut (as described elsewhere in this disclosure) and, thereby, reduce target site deformation, penetration member 168 easily penetrates the target site to a controlled depth and with reduced discomfort to a user in comparison to sampling modules that do not include such a floating ring. Once apprised of the present disclosure, one skilled in the art will recognize that various conventional trigger mechanisms can be employed to initiate the launching of penetration member 168.

Pressure ring 170 is attached to outer shaft 152 (see FIGs. 7 and 8) and is, therefore, placed in a retracted or deployed state by movement of spring retainer 156 in response to rotation of cam 126. Pressure ring 170 can be attached to outer shaft 152 by any suitable means including, for example, by frictional engagement. One skilled in the art will recognize that cam 126, spring retainer 156 and outer shaft 152 are configured as a pressure ring retraction and deployment mechanism. However, one skilled in the art will also recognize that other suitable mechanical assemblies can also be employed for the same purpose.

Sampling module 100 is configured for attachment to a user's body (B), for example, a user's forearm. Such configuration includes pins 144 mounted to plate 138 by brackets 142 and screws 148 (see FIG. 2). A rigid or elastic arm band (not shown) is attached to pins 144 such that sampling module 100 can be securely and removably attached to the user's body.

When sampling module 100 is in a retracted state (see FIG. 7), pressure ring 170 is applying essentially zero pressure to the user's body in the vicinity of the target site (i.e., a first vicinity) even though penetration member 168 has been launched and penetrated the target site. Pressure ring 170 is in a retracted state since cam 126 has been rotated clockwise, thereby lifting roller 134s and spring retainer 156. In this retracted state, rollers 134 are in locked positions within helical groove 128 of cam 126. When cam 126 has been rotated clockwise such that roller 134s are in such a locked position, pressure springs 160 do not apply any force to pressure ring 170. In this retracted state, spring retainer 156 is in an uppermost position within housing assembly 102, causing springs 160 to be fully compressed. Such a compression of springs 160 prevents springs 160 from pushing downward on shaft step 154.

In the retracted state, pressure ring 170 is floatably attached to housing assembly 102 such that the only pressure applied in the vicinity of the target site by the pressure ring is solely related to the mass of the pressure ring and mass transferred from associated components (e.g., inner shaft 150 and outer shaft 152) of the sampling module (referred to collectively as essentially zero pressure). In other words, the pressure applied by the pressure ring in a retracted state is solely related to the mass of the pressure ring assembly and housing assembly. Furthermore, floating ring 172 also does not apply any pressure in the vicinity of the target site due to its floating nature (other than a negligible pressure due to the mass of the floating ring and pressure from the mass of other sampling components that is effectively transferred by friction through the floating ring). In the retracted state, therefore, only a minimal pressure is applied to the user's body in the vicinity of the target site, thus maximizing recovery of the target site and vicinity while the sampling module is in a retracted state. Such recovery has been determined to be beneficial in terms of maximizing the overall time period over which ISF samples can be successfully extracted at adequate flow rates for analyte determination and in terms of mitigating lag between analyte ISF concentration and analyte blood concentration.

When sampling module 100 is in a deployed state (see FIG. 8), penetration member 168 has been previously launched, penetrated the target site and is residing in the target site. Penetration member 168 can reside, for example, at a penetration depth in the range of about 1.5mm to 3mm below the surface of the target site (e.g., below the surface of a user's target site skin layer). If desired, penetration member 168 may be launched coincidentally with an initial placement of pressure ring 170 into the deployed state illustrated in FIG. 8.

When sampling module 100 is in a deployed state (again, see FIG. 8), pressure ring 170 applies pressure in a first vicinity of the target site that serves to pressurize ISF in that first vicinity. A sub-dermal pressure gradient induced by pressure ring 170 results in flow of ISF through penetration member 168 and to an analysis module (not shown) for determining an analyte in the ISF (such as glucose). Further details regarding the use of pressure rings for the extraction of ISF, albeit in the absence of a floating pressure ring with adhesive layer and the deployment and retraction mechanisms described herein, are in U.S. Patent Application Nos. 10/652,464 (published as US 2004/0253736), 10/653,023 (published as US 2004/0249253) and 10/861,749 (published as US 2004/0249254), which are hereby incorporated in full by reference.

ISF flow rate through a penetration member is subject to potential reduction over time due to depletion of ISF in the vicinity of the target site and/or due to relaxation of the target site, despite the presence of pressure ring 170, when sampling module 100 is in a deployed state. However, the presence of floating ring 172 adhered at a second vicinity of the target site (see, for example, FIG. 8) serves to reduce target site relaxation and, thus, minimize deleterious reduction in ISF flow rate. Moreover, potential reduction in ISF flow rate can be minimized by oscillating sampling module 100 between the deployed state of FIG. 8 and the retracted state of FIG. 7.

In the deployed state, the pressure (force) applied in the first vicinity of the target site by pressure ring 170 can be, for example, in the range of from about 1 pound per square inch to about 150 pounds per square inch (PSI, calculated as force per area of pressure ring 54 contacting the user's body), and more typically in the range from about 30 PSI to about 70 PSI. In this regard, a pressure of approximately 50 PSI has been determined to be beneficial with respect to providing adequate ISF flow while minimizing user pain/discomfort.

In the deployed state of FIG. 8, cam 126 is rotated counter-clockwise to the full extent possible such that roller 134 has fixedly and reversibly engaged another locking position of helical groove 128. An effect of this counter-clockwise rotation of cam 126 is that cam 126 has moved upward causing a gap of dimension D5 between cam 126 and plate 138 (see FIG. 8). When cam 126 disengages from plate 138, pressure springs 160 apply force to pressure ring 170 (in the manner previously described).

In the retracted state, the aforementioned gap between cam 126 and plate 138 will have a dimension approaching zero (see FIG. 7) and outer E-ring 116 will be touching cap 118. Outer E-ring 116 is mounted to outer shaft 152 and, therefore, serves to limit the maximum downward movement of outer shaft 152 because outer E-ring 116 is bounded by cap 118.

As is evident from a comparison of FIGs. 7 and 8, floating ring 172 essentially moves with pressure ring 170 as pressure ring 170 is moved from a retracted state to a deployed state.

Once apprised of the description herein related to FIGs. 1-8, one skilled in the art will recognize that, in general, a sampling module for extracting interstitial fluid (ISF) from a user's body according to another embodiment can include a housing assembly and a pressure ring assembly operatively contained within the housing assembly. The pressure ring assembly of such an embodiment includes a penetration member (e.g., a needle) configured for penetrating a target site of the user's body and, subsequently, residing in the target site and extracting ISF therefrom. The pressure ring assembly also includes a pressure ring configured for applying pressure to the user's body in a first vicinity of the target site and a floating ring with an adhesive layer disposed on the distal end of the floating ring. The adhesive layer of such a sampling module provides for adhesive attachment of the floating ring to the user's body in a second vicinity of the target site, thereby influencing target site deformation during use of the sampling module.

Sampling modules according to embodiments of the present invention provide several benefits. First, the presence of a floating ring with an adhesive layer provides beneficial control (i.e., a beneficial influence) over deformation of a target site during application of pressure by the floating ring. Since the floating ring is "floating" with respect to movement in the vertical direction, there is no biasing downward pressure being applied in the vicinity of the target site by the floating ring. However, the adhesive layer of the floating ring holds the floating ring on the user's body, thus placing the target site under tension as the pressure ring applies pressure. This tension beneficially reduces the required travel of the pressure ring and, thus, the size of the sampling module.

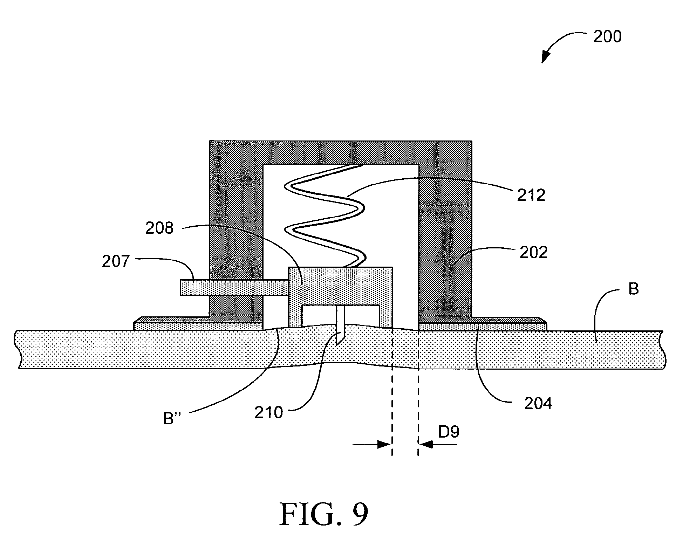

FIGs. 9-11 depict, in a simplified manner the benefits of a floating pressure ring with adhesive layer that is employed in various embodiments of the present invention.

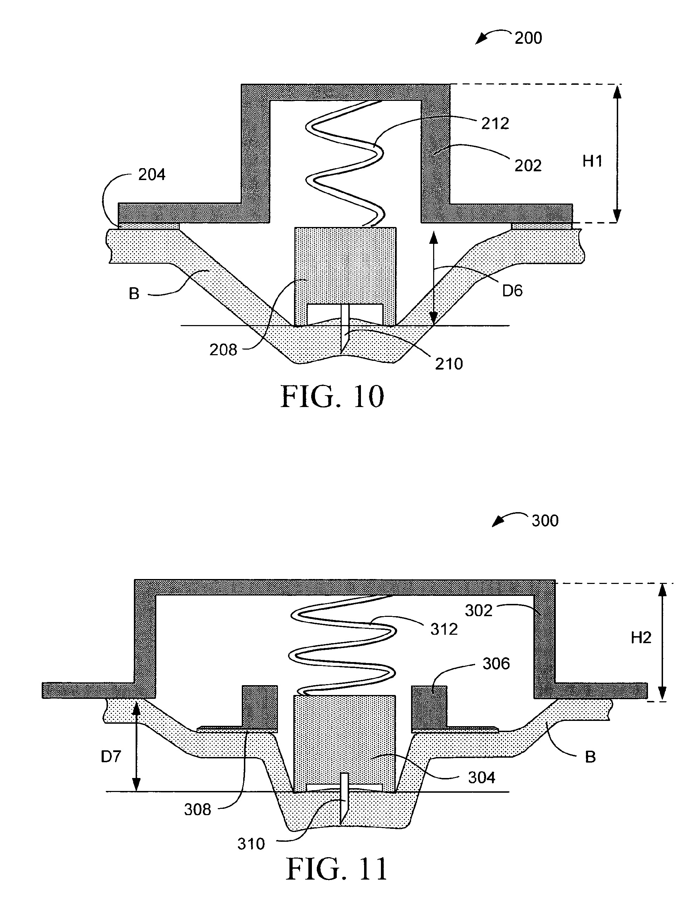

FIG. 9 is a simplified cross-sectional view of a sampling module 200 for extracting ISF, which does not include a floating ring, in a retracted state and attached to a user's body B. Sampling module 200 includes a housing assembly 202 (with adhesive layer 204), a spring 212 (shown in FIG. 10), a pressure ring 208, a biasing member 207, and a penetration member 210. Sampling module 200 is secured user's body B (i.e., a user's skin layer) using adhesive layer 204 and an armband (not shown). Although pressure ring 208 is in a retracted state, a portion of the user's body (B") has deformed such that pressure ring 208 is applying pressure despite being in a retracted state. It is hypothesized without being bound that housing assembly 202 is itself applying pressure to the user's body at a distance D9 from pressure ring 208 that causes deformation of the user's body and affects ISF extraction rates.

FIG. 10 is a simplified cross-sectional view of sampling module 200 of FIG. 9 in a deployed state with spring 212 of sampling module 200 causing pressure ring 208 to apply a pressure to user's body B, thus deforming user's body B by a relatively large distance D6. To place sampling module 200 in the deployed state, biasing member 207 is removed, thereby allowing spring 212 to apply a downward force onto pressure ring 208. The downward force applied by pressure ring 208 is offset by an opposing upward force by housing assembly 202. Deformation of user's body B, results in pressure ring 208 having a relatively large travel and, thus, sampling module 200 is relatively large in size (i.e., height H1 of FIG. 10). However, deformation of user's body B can be minimized by making distance D9 relatively small, for example, less than about 2 millimeters.

Based on the discussion above and FIGs. 9 and 10, sampling modules without the floating ring employed in embodiments of the present invention can suffer from inadvertent application of pressure when in a retracted state and excessively large deformation of the user's body in the deployed state. Moreover, such excessively large deformation can lead to decreased ISF extraction rates over time.

FIG. 11 is a simplified cross-sectional view of a sampling module 300 for extracting ISF according to another exemplary embodiment of the present invention in a deployed state in a user's body B. Sampling module 300 includes a housing assembly 302 (with a housing assembly adhesive layer, not shown), a pressure ring 304, a floating ring 306 (with adhesive layer 308), a penetration member 310 and a spring 312. In the embodiment illustrated in FIG. 11, floating ring 306 and adhesive layer 308 serve to hold the user's body taut (i.e., create tension in the user's body) in the vicinity of a target site (that has been penetrated by penetration member 310) as pressure ring 304 is applying pressure. This tension serves to limit pressure ring travel (distance D7 in FIG. 11) while still providing for pressure ring 304 to apply pressure in the vicinity of the target site.

The reduction in pressure ring travel and sampling module size (i.e., height) in sampling modules according to the present invention is a function of the pressure applied by the pressure ring and other dimensions and characteristics of the sampling module. However, if it is assumed that the respective springs 212 and 312 (see FIGs. 10 and 11) have the same force constant and compressibility characteristics, then pressure ring 208 must travel a greater distance D6 than the distance D7 traveled by pressure ring 304. Thus, floating ring 306 with adhesive layer 308 provides for pressure ring 304 to efficiently apply pressure with a smaller travel and less deformation of the user's body. In this regard, dimension H2 can be less than dimension H1, resulting in a sampling module of smaller size and less discomfort during use. For example, while H1 can be in the range of 20mm to 40mm, H2 can be in the range of 10mm to 15mm.

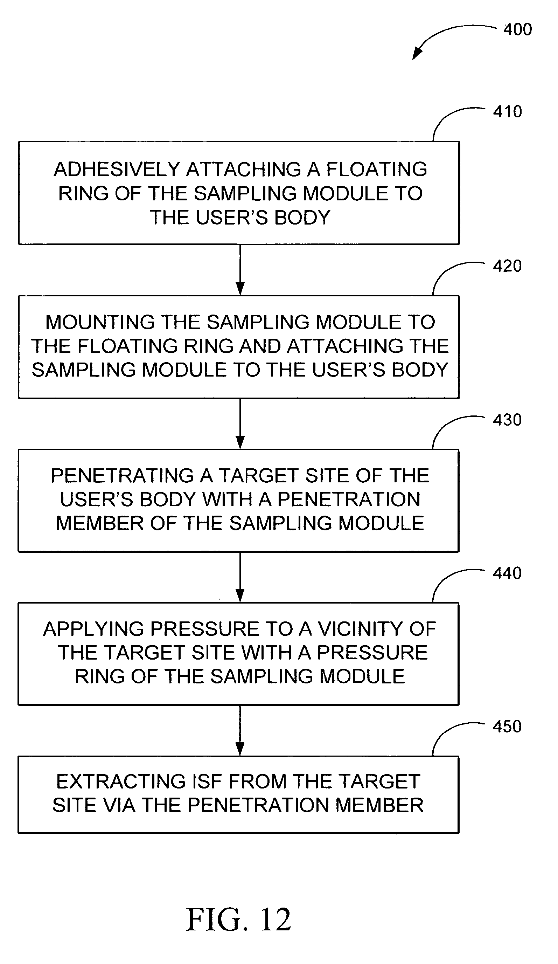

FIG. 12 is a flow diagram depicting stages in a method 400 for extracting interstitial fluid from a user according to an exemplary embodiment of the present invention. Method 400 includes adhesively attaching a floating ring of a sampling module to a user's body in a vicinity of the target site (e.g., a second vicinity as described above), as set forth in step 410. The sampling module thus attached can be any sampling module according to the present invention as described herein.

Subsequently, at step 420, a sampling module for extracting interstitial fluid from a user's body to the user is mounted onto the attached floating ring and attached to the user's body. The sampling module can be mounted on the floating ring such that the sampling module is, for example, concentrically disposed about the floating ring. Optionally, the sampling module can include rids that are employed to slidingly secure the floating ring to the sampling module as was described above with respect to sampling module 100.

A target site of the user's body is then penetrated with a penetration member of the sampling module, as set forth in step 430. Pressure is then applied in another vicinity of the target site (e.g., the first vicinity described above) by a pressure ring of the sampling module and, thereafter, ISF is extracted from the target site via a penetration member of the sampling module (see steps 440 and 450).

Exemplary mechanics by which steps 410 through 450 can be accomplished have been described above with respect to FIGs. 1-11. For example, the pressure ring can apply the pressure by being placed in the deployed state described above. In addition and if desired, the sampling module can be cycled between a deployed state and a retracted state for an extended period of time (e.g., 8 hours or more) to facilitate semi-continuous extraction of ISF. The deployed state can have a duration of, for example, 5 minutes while the retracted state can have a duration of, for example, 10 minutes. Such durations have been determined to provide a beneficial ISF extraction rate of 50nL/minutes during the deployed state for periods of at least eight hours.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A sampling module for extracting interstitial fluid (ISF) from a user's body, the sampling module comprising:
a housing assembly; and
a pressure ring assembly operatively contained within the housing assembly, the pressure ring assembly including:
a penetration member configured for penetrating a target site of a user's body and, subsequently, residing in the target site and extracting ISF therefrom;
at least one pressure ring configured for applying pressure to the user's body in a first vicinity of the target site while said penetration member is residing the target site; and
at least one floating ring that includes:
a proximal end;
a distal end; and
an adhesive layer disposed on the distal end of the floating ring; wherein the adhesive layer provides for adhesive attachment of the floating ring to the user's body in a second vicinity of the target site, thereby influencing target site deformation during use of the sampling module.

2. The sampling module of claim 1, wherein the first vicinity where the pressure ring applies pressure closer to the target site than the second vicinity where the floating ring is adhesively attached to the user's body.

3. The sampling module of claim 1, wherein the floating ring influences target site deformation by placing the target site under tension when the pressure ring is applying pressure in the first vicinity of the target site.

4. The sampling module of claim 1 further including a pressure ring retraction and deployment mechanism that serves to place the pressure ring in a retracted state wherein the pressure applied to the first vicinity by the pressure ring is related solely to the mass of the pressure ring assembly and housing assembly.

5. The sampling module of claim 4, wherein the pressure ring retraction and deployment mechanism includes a cam operatively engaged with the pressure ring.

6. The sampling module of claim 5, wherein the floating ring applies a pressure to the second vicinity, when the pressure ring is in a retracted state, that is solely related to the mass of the floating ring and pressure related to mass of the sampling module that is transferred to the second vicinity via the floating ring.

7. The sampling module of claim 1, wherein the pressure ring has an opening therethrough, an inner diameter in the range of from about 4mm to about 12mm and an outer diameter in the range of from about 5mm to about 13mm.

8. The sampling module of claim 1, wherein the floating ring has an opening therethrough, an inner diameter in the range of from about 7mm to about 18mm and an outer diameter in the range of from about 33mm to about 50mm.
